# EUROPÄISCHE PATENTSCHRIFT

(11) **EP 0 360 951 B1**
(45) Veröffentlichungstag und Bekanntmachung des Hinweises auf die Patenterteilung: **01.09.1993**
(21) Anmeldenummer: 89101057.1
(22) Anmeldetag: 21.01.1989
(51) Int. Cl.: A61B 19/00

(54) **Einmal-Überzug aus einer Schlauchfolie für Arthroskopiekameras und Verfahren zu dessen Verwendung**
Disposable thin-walled tube covering for an arthroscopy camera, and use thereof
Enveloppe à usage unique en forme de manche mince pour appareil photo à arthroscopie, et son mode d'emploi

(30) Priorität: 23.09.1988 DE 8812027 U; 15.12.1988 DE 8815549 U
(43) Veröffentlichungstag der Anmeldung: 04.04.1990
(73) Patentinhaber: Dunsch-Herzberg, Renate, D-22880 Wedel (DE); Voss, Gudrun, D-25491 Hetlingen (DE)
(72) Erfinder: Herzberg,Wolfgang,Dr.med., D-2000 Wedel/Holstein (DE)
(74) Vertreter: Richter, Werdermann & Gerbaulet

(56) Entgegenhaltungen:
- DE-U- 8 711 189
- US-A- 3 698 791
- US-A- 4 522 196
- US-A- 4 649 910

## Beschreibung

Die Erfindung betrifft einen Einmal-Überzug aus einer Schlauchfolie für Arthroskopiekameras und ein Verfahren zum Abdecken von Arthroskopiekameras mittels eines derartigen Einmal-Überzuges.

Ausgelöst durch die Einführung von Video-Kameras in die Technik der Gelenkspiegelung entstand die Notwendigkeit, eine unsterile, kabeltragende Kamera durch einen sterilen Folienüberzug dem Chirurgen zugänglich zu machen.In der ersten Generation dieser Überzüge standen einfach gelegte Schlauchfolien aus Polyäthylen zur Verfügung, die in relativ mühsamer Weise über die Kamera geschoben werden. In der zweiten Generation wurden diese Überzüge mit einer sogenannten "Steckfaltung"entwickelt, d.h., der Folienschlauch war mit mehreren Abschnitten teleskopartig eingezogen und konnte somit einfach über die Kamera gezogen werden.

Sowohl die "einfach" gelegte als auch die "gesteckte" Schlauchfolie benötigen zum Verschluß der Schlauchenden Klebestreifen,
- da das "sterile" Ende des Schlauches unter Umständen einer erheblichen Befeuchtung ausgesetzt ist und somit die Gefahr einer Ablösung des Klebestreifens besteht;
- die räumlich äußerst bemessene Anordnung von zu-und ableitenden Kabelanschlüssen an dem sterilen Ende des Schlauches läßt das Anbringen eines breiten Klebestreifens nicht zu.

Hinzu kommt, daß die bekannten Schlauchfolien an ihrem sterilen Ende eine Verjüngung des Schlauches aufweisen, die durch zwei geschweißte,konvergierende Abtrennungen erzeugt wird. Diese beiden Schweißnähte liegen in dem stärksten durch Manipulation beanspruchten Bereich und stellen eine erhebliche Gefahrenzone bezüglich der Sterilitätsverletzung dar. Ein Verzicht auf diese Abnähte würde jedoch dazu führen, daß der Verschluß durch den Klebestreifen einen gewissen "Konfekttüten" -Effekt mit einer störenden Krempenbildung erzeugt.

Mit der Sterilhaltung und Neusterilisierung von medizinischen Instrumenten, wie Spritzen, Kanülen oder sonstigen Instrumenten sowie Verbandstoffen in Kunststoffbeuteln befaßt sich die DD-Z. Zschr.ärztl.Fortbild 60.Jg. H.9, S.581-583, in der davon ausgegangen wird, daß nach den bisher bekannten Methoden eine Sterilhaltung von Spritzen für längere Zeit nicht immer gewährleistet sein würde und daß chirurgische Instrumentarien in Ärztekoffern häufig in Leinentaschen verpackt sein würden, so daß die Sterilität damit fragwürdig sein würde. Derartige chirurgische Instrumente sollen daher für die Soforthilfe nicht einsetzbar sein. Aus diesem Grunde wird vorgeschlagen, medizinische Instrumente in Plastikbeuteln zu verpacken, so daß dann die in Plastikfolien verpackten Instrumente und Verbandstoffe an die einzelnen Facheinrichtungen oder Einrichtungen im Versorgungsgebiet ausgeliefert werden können, wobei dann gewährleistet sein soll, daß insbesondere in beweglichen Rettungseinrichtungen das Instrumentarium sicher sterilisert in einer gewichtslosen Verpackung untergebracht ist.

Die GB-A-10 53 955 beschreibt eine Folienverpackung aus einer Folienhülle, die mehrlagig ausgebildet und in gerollter Form eingesetzt wird. Eine derartige Verpackung ist nicht als Einweg-Überzug für Arthroskopiekameras einsetzbar. Auch ist hierbei keine Unterbringung eines Schlauchfolienvorrates in einem geschlossenen Raum vorgesehen, dem bei Bedarf der Schlauchfolienvorrat entnommen werden kann.

Eine Folienabdeckung für ein an einem auskragenden Schwenkarm angeordneten Operationsmikroskop, wobei die Folienabdeckung als Folienhülle ausgebildet ist, ist durch die US-A-36 98 791 bekannt. Die Abdeckung des Schwenkarmes mit dem Operationsmikroskop erfolgt dabei durch Aufziehen der Folienhülle über die gesamte Einrichtung, wobei die Folienhülle einendig verschlossen ausgebildet ist, während das andere offene Ende der Folienhülle nach dem Aufziehen auf das Operationsmikroskop und seinen Schwenkarm mittels eines Schlauchbinders verschlossen wird. Vor Gebrauch ist die Folienhülle mehrfach gefaltet und paketartig zusammengelegt. Im Bereich des verschlossenen Schlauchfolienendes sind zwei fingerartig ausgebildete Ausstülpungen vorgesehen, die, wenn die Folienhülle über das Operationsmikroskop und seinen Schwenkarm gezogen wird, die beiden Okulare des Mikroskopes umhüllen.

Der Erfindung liegt daher die Aufgabe zugrunde, einen Einmal-Überzug aus einer Schlauchfolie für Arthroskopiekameras und ein Verfahren zum Abdecken von Arthroskopiekameras mittels eines derartigen Einmal-Überzuges zu schaffen, wonach die Handhabung eines derartigen Einmal-Überzuges einfach und schnell ist, wobei eine erhöhte Sicherheit in der Problemzone erreicht wird und es sich keine Schwachstellen durch Schweißnähte ergeben bei gleichzeitiger Erhöhung der Sterilität.

Diese Aufgabe wird bei einem Einmal-Überzug der eingangs genannten Art durch die im Patentanspruch 1 gekennzeichneten Merkmale gelöst.

Des weiteren wird die Aufgabe durch ein Verfahren zum Abdecken von Arthroskopiekameras mit einem derartigen Einmal-Überzug mit den im Patentanspruch 7 bzw. 8 angegebenen Merkmalen gelöst.

Bei einem derart ausgebildeten Einmal-Überzug entfallen an dem arthroskopseitigen Schlauchende Schweißnähte, Perforationen und Klebestreifen, da Schlauchbinder eingesetzt werden, die in ihrer Handhabung sicherer und akurater gegenüber Klebestreifen und zudem noch gegen Feuchtigkeit völlig unempfindlich sind. Der Einmal-Überzug ist einfach und schnell zu handhaben. Eine doppelte Sicherheit ist durch die zweifache Folienlage in der Problemzone gegeben. Der Verschluß ist feuchtigkeitssicher. Es ist keine Sterilitätsgefährdung durch Schweißnähte gegeben. Die Fixierung des Kabels ist sowohl im Operationsfeld als auch am Monitor komfortabler.

Besonders vorteilhaft ist dabei die Ausgestaltung nach Anspruch 5, nach der das Faltpaket als Schlauchfolienspeicher in einem Behältnis untergebracht ist Ein derart ausgebildeter Einmal-Überzug aus einer Schlauchfolie ermöglicht die Unterbringung einer ziehharmonikaartig gefalteten Schlauchfolie in einem scheibenförmigen Behälter mit Durchbrechungen in den sich gegenüberliegenden Behälterwänden, um die beiden Enden der speziell gefalteten Schlauchfolie aus dem Innenraum des Behälters herausziehen zu können und um das Arthroskop durch den Behälterinnenraum in den Innenraum des einen Schlauchfolienabschnittes einführen zu können Die Anordnung der Schlauchfolie ist dabei so getroffen, daß die Folienfaltung im Innenraum des Behälters zu liegen kommt, während das eine Ende der Schlauchfolie über das andere Ende, und zwar in paralleler Richtung zur Schlauchfolie, gezogen werden kann, wobei Schlauchfolienmaterial aus der Schlauchfolienfaltung entnommen wird. Ist ein Arthroskop durch den Innenraum des Schlauchfolienaufnahmebehälters hindurchgeführt und in den inneren Schlauchabschnitt eingeführt, dann erfolgt im Bereich des Arthroskopes die Abbindung der Schlauchfolie vermittels eines Schlauchbinders, worauf in das freie Ende des inneren Schlauchfolienabschnittes die unsterile Kamera eingeführt wird, woraufhin dann der äußere Endabschnitt der Schlauchfolie über die innenliegende Schlauchfolie gezogen wird, so daß nach vollständiger Entfaltung der Schlauchfolie der leere Behälter zurückbleibt, wohingegen die Kamera dann zweifach von Folie umhüllt ist.

Aufgrund der erfindungsgemäßen Ausgestaltung ist die Verwendung eines Einmal-Überzuges aus einer Schlauchfolie möglich, der keine Schwachstellen durch Schweißnähte aufweist. Der Überzug stellt sicher,daß die durch Manipulation gefährdete Sterilität am sterilen Ende durch eine doppelte Folienlage zweifach gesichert wird. In seiner Handhabung entspricht der Überzug dem Komfort der bekannten Steckfaltung. An seinem sterilen Ende weist der Überzug einen absolut feuchtigkeitssicheren Verschluß auf. Die eng bemessenen Verhältnisse am sterilen Ende des Überzuges sind nicht durch einen räumlich sparsamen Verschluß störend überfordert. Sowohl in Schlauchmitte als auch am unsterilen Ende ist eine sichere Befestigung und ein sicherer Verschluß möglich. Durch die Anordnung des gefalteten Abschnittes der Schlauchfolie in dem Innenraum des Behälters ist eine sichere Aufbewahrung und eine einfache Handhabung der Schlauchfolie bei Aufrechterhaltung einer hohen Sterilität gewährleistet.

Das Verfahren zum Abdecken von Arthroskopiekameras mit einem Einmal-Überzug besteht nach der Erfindung darin, daß das freie Ende des außenliegenden Abschnittes einer zu einem Faltlagenpaket mit parallel zur Schlauchfolienlängsrichtung verlaufenden Faltlagen zusammengefalteten, sterilen Schlauchfolie um einen Abschnitt vom Faltlagenpaket abgezogen und über den innenliegenden, aus dem Faltlagenpaket geringfügig abgezogenen Abschnitt der Schlauchfolie hinweggezogen, hierauf in den Innenraum des innenliegenden Abschnittes der Schlauchfolie das Arthroskop eingeschoben, die Schlauchfolie im Bereich des freien Schlauchfolienendes hinter der Optik des Arthroskopes abgebunden, danach der außenliegende Abschnitt der Schlauchfolie unter Verwendung des Schlauchfolienvorrates des Faltlagenpaketes über die auf die Optik des Arthroskopes aufgesetzte Kamera bis sich der innenliegende Schlauchfolienabschnitt im Abbindungsbereich um die Optik des Arthroskopes stülpt zur Ausbildung einer zweilagigen Folienabdeckung gezogen und das freie Ende der abgezogenen Schlauchfolie hinter der Kamera abgebunden wird. Hiernach wird ein Schlauchfolienvorrat verwendet, der aus zahlreichen , aus der Schlauchfolie gebildeten Faltlagen besteht, die parallel zur Längsrichtung der Schlauchfolie verlaufend sind.

Ferner sieht die Erfindung ein Abdeckverfahren von Arthroskopiekameras mit einem Einmal-Überzug vor, das darin besteht, daß das freie Ende des außenliegenden Abschnittes einer zu einem in einem geschlossenen Raum angeordneten Faltlagenpaket mit quer zur Schlauchfolienlängsrichtung verlaufenden Faltlagen zusammengefalteten sterilen Schlauchfolie um einen Abschnitt vom Faltlagenpaket abgezogen, der innenliegende Abschnitt der Schlauchfolie vom Faltlagenpaket mit einem gegenüber der Länge des äußeren Schlauchfolienabschnittes größeren Abschnitt abgezogen, hierauf in den Innenraum des inneren Schlauchfolienabschnittes das Arthroskop eingeschoben, die innere Schlauchfolie im Bereich der Optik des Arthroskopes abgebunden, danach in den inneren Schlauchfolienabschnitt durch dessen Öffnung am freien Ende die Kamera eingeschoben, darauf der äußere Schlauchfolienabschnitt unter Verwendung des Schlauchfolienvorrats des Faltlagenpaketes über die auf die Optik des Arthroskopes aufgesetzte Kamera und den inneren Schlauchfolienabschnitt bis sich der innenliegende Schlauchfolienabschnitt im Abbindungsbereich um die Optik des Arthroskopes stülpt zur Ausbildung einer zweilagigen Folienabdeckung gezogen und das freie Ende der abgezogenen Schlauchfolie hinter der Kamera abgebunden wird.

Weitere vorteilhafte Ausgestaltungen der Erfindung sind in den abhängigen Ansprüchen gekennzeichnet.

Ausführungsbeispiele der Erfindung werden nachstehend anhand der Zeichnung erläutert. Es zeigt
Fig. 1 in einer schaubildlichen Ansicht einen Einmal-Überzug mit einem Schlauchfolienspeicher aus einem Faltlagenpaket aus mehreren parallel zur Schlauchfolienlängsrichtung verlaufenden Faltlagen,
Fig. 2 eine Seitenansicht einer weiteren Ausführungsform des Einmal-Überzuges mit einem anderen Faltlagenverlauf,
Fig. 3 eine Seitenansicht des Einmal-Überzuges mit dem Schlauchfolienspeicher,
Fig. 4 eine Seitenansicht des Einmal-Überzuges nach Fig 1 während des Einführens des Arthroskops,
Fig. 5 eine Seitenansicht des Einmal-Überzuges nach Fig 1 in der Schlauchfolienabziehphase,
Fig. 6 teils in Ansicht, teils in einem senkrechten Schnitt einen Einmal-Überzug mit einem in seinem Behälter angeordneten Schlauchfolienspeicher aus einem Faltlagenpaket aus mehreren quer zur Schlauchfolienlängsrichtung verlaufenden Faltlagen,
Fig. 7 eine Seitenansicht der Faltung bzw. Raffung der Schlauchfolie des Einmal-Überzuges nach Fig 6,
Fig. 8 eine Seitenansicht des Einmal-Überzuges nach Fig 6 mit dem Schlauchfolienspeicher,
Fig. 9 in einer schaubildlichen Ansicht den Einmal-Überzug mit dem in einem Behälter angeordneten Schlauchfolienspeicher mit in den inneren Schlauchabschnitt der Schlauchfolie eingeführtem Arthroskop,
Fig. 10 in einer schaubildlichen Ansicht den Einmal-Uberzug mit dem in einem Behälter angeordneten Schlauchfolienspeicher mit in den inneren Schlauchabschnitt der Schlauchfolie eingeführter und auf das Arthroskop aufgesetzter Kamera,
Fig. 11 in einer schaubildlichen Ansicht den Einmal-Überzug mit dem in einem Behälter angeordneten Schlauchfolienspeicher mit zweifach von Schlauchfolie umhüllter Kamera und
Fig. 12 in einer schaubildlichen Ansicht den Einmal-Überzug mit dem in einem Behälter angeordneten Schlauchfolienspeicher mit zweifach von Schlauchfolie umhüllter Kamera und seitlich entferntem Behälter.

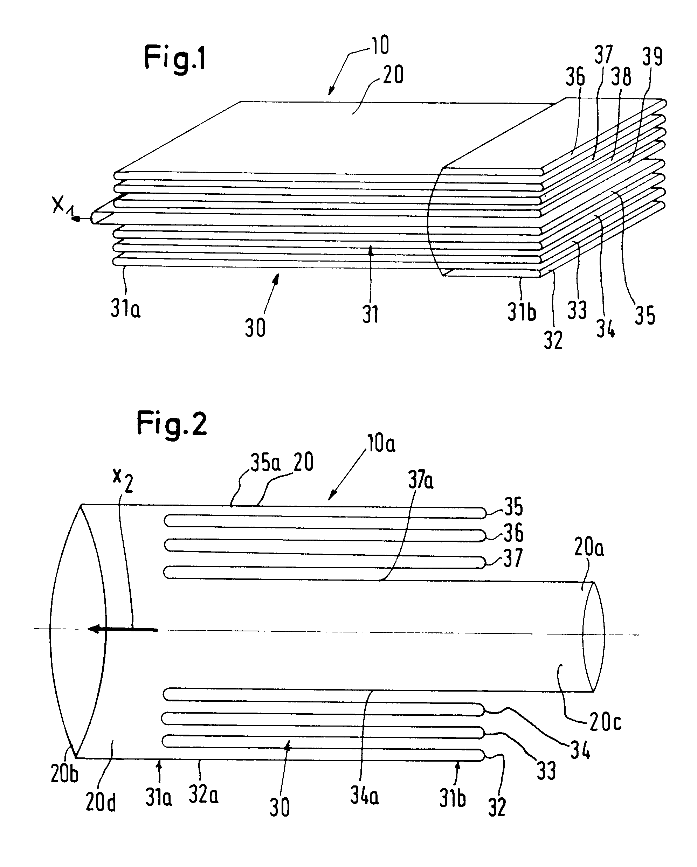

Nach Fig 1 besteht der Einmal-Überzug 10 für Arthroskopiekameras aus einer Schlauchfolie 20 und weist einen aus der Schlauchfolie 20 gebildeten Schlauchfolienspeicher 30 auf, der von einer Anzahl von Faltlagen 32,33,34,35,36,37,38,39 der Schlauchfolie 20 gebildet wird, die zu einem Faltlagenpaket 31 zusammengefaßt sind, dessen Endbereiche bei 31a, 31b angedeutet sind.

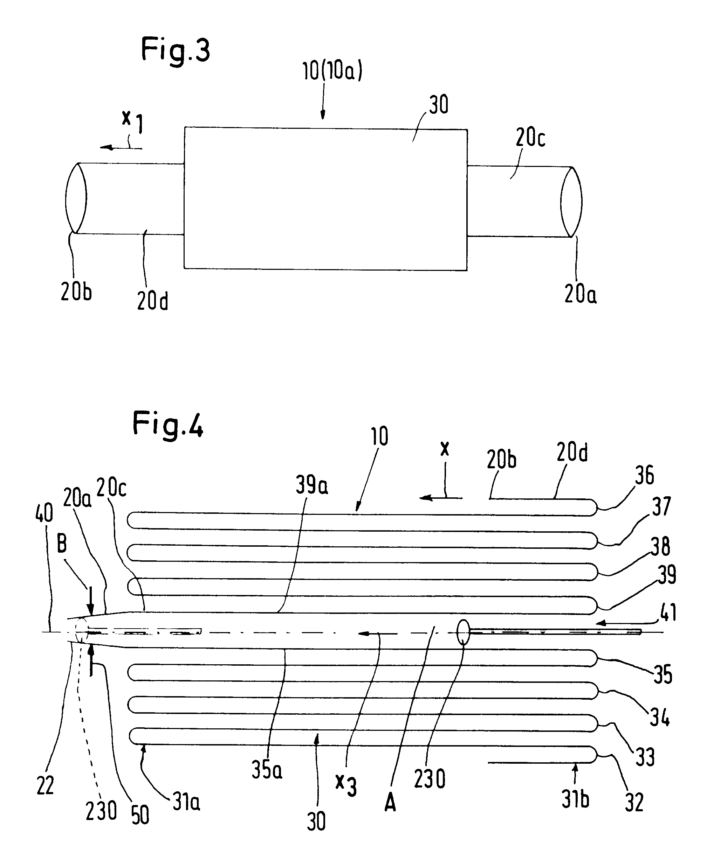

Bei diesem Einmal-Überzug 10 verlaufen die Faltlagen 32 bis 39 parallel zur Schlauchfolienlängsrichtung, die in Fig 4 bei 40 angedeutet ist. Bei dem in Fig. 1 gezeigten Ausführungsbeispiel weist das Faltlagenpaket 31 acht aus der Schlauchfolie 20 gebildete Faltlagen 32 bis 39 auf, wobei die Anzahl der Faltlagen auch geringer oder größer sein kann Die Faltung der Schlauchfolie 20 zum Faltlagenpaket 31 erfolgt in der sogenannten Steckfaltung, die es ermöglicht, daß beim Abziehen des freien Endes der beiden äußeren Faltlagen 32,36 in Pfeilrichtung X durch Auflösen der Faltlagen die gesamte Schlauchfolie 20 abgezogen werden kann. Die beiden Enden der Schlauchfolie 20 sind mit 20a,20b bezeichnet, wobei 20a das Schlauchfolienende des inneren Schlauchfolienabschnittes 20c ist, während 20b das Schlauchfolienende des äußeren Schlauchfolienabschnittes 20d ist (Fig.4).

Zwischen den beiden inneren Faltlagen 35,39 ist eine Einführöffnung 41 für ein Arthroskop 30 ausgebildet, wobei diese Einführöffnung 41 von den beiden gegenüberliegenden Schlauchfolienabschnitten 35a,39a der beiden Faltlagen 35,39 begrenzt ist, wobei diese Folienabschnitte 35a,39a den inneren Schlauchfolienabschnitt 20c bilden.

Dieser innere Schlauchfolienabschnitt 20c ist aus dem Faltlagenpaket 31, und zwar aus dessen Endbereich 31a, herausgeführt, und zwar unter Ausbildung eines herausragenden Abschnittes 22 (Fig. 4).

Bei 50 ist in Fig 4. ein in an sich bekannter Weise ausgebildeter Schlauchbinder angedeutet, worauf nachstehend noch näher eingegangen wird.

Fig. 3 zeigt die Schlauchfolie 20 des Einmal-Überzuges mit dem Schlauchfolienspeicher 30, der aus den Faltlagen 32 bis 39 gebildet wird. Für den Gebrauch des Einmal-Überzuges 10 und zur Ausbildung einer zweifachen Folienlage im Überzugsbereich des Arthroskopes und der dazu erforderlichen Kamera wird entweder der Schlauchfolienabschnitt 20d mit seinem Schlauchfolienende 20b in Pfeilrichtung X1 über den Schlauchfolienabschnitt 20c oder durch den Innenraum des Schlauchfolienabschnittes 20c soweit geführt und gezogen, daß in der Endphase der herausragende Schlauchfolienabschnitt 22 ausgebildet ist.

Bei dem Einmal-Überzug 10a nach Fig.2 ist die Schlauchfolie 20 gegenüber der Faltlage bei dem Einmal-Überzug 10 entsprechend Fig.1 anders gestaltet. Dieser Einmal-Überzug 10a weist ebenfalls einen Schlauchfolienspeicher 30 auf, der von den Faltlagen 32,33,34,35,36,37 gebildet wird, die das Faltlagenpaket 31 darstellen. Bei diesem Einmal-Überzug 10a bilden die verlängerten Faltlagen 34,37 mit ihren verlängerten Abschnitten 34a,37a die Wandflächen bzw. die umlaufende Wandfläche des inneren Schlauchfolienabschnittes 20c, während der äußere Schlauchfolienabschnitt 20d von den äußeren Folienabschnitten 32a,35a der Faltlagen 32,35 gebildet wird. Auch wenn die Schlauchfolienenden 20a,20b in den beiden Endbereichen 31a,31b des Faltlagenpaketes 31 liegen, so wird doch für den Gebrauch des Einmal-Überzuges 10a der innere Schlauchfolienabschnitt 20c in Pfeilrichtung X2 aus dem Ende 20b des äußeren Schlauchfolienabschnittes 20d soweit herausgeführt, daß der herausragende Abschnitt 22 ausgebildet ist. Auch bei dem Einmal-Überzug 10a wird der Schlauchfolienspeicher 30 von einer Anzahl Faltlagen nach Art der Steckfaltung gebildet. Sowohl beim Einmal-Überzug 10 als auch beim Einmal-Überzug 10a kann die Anzahl der Faltlagen beliebig sein und wird sich jeweils nach der Länge der eingesetzten Schlauchfolie 20 richten, was letztlich auch von der Länge einer jeden Faltlage abhängt Das Faltlagenpaket 31 des Einmal-Überzuges 10,10a kann z.B. vier oder auch mehr Faltlagen 32 bis 39 bzw. 32 bis 35 aufweisen.

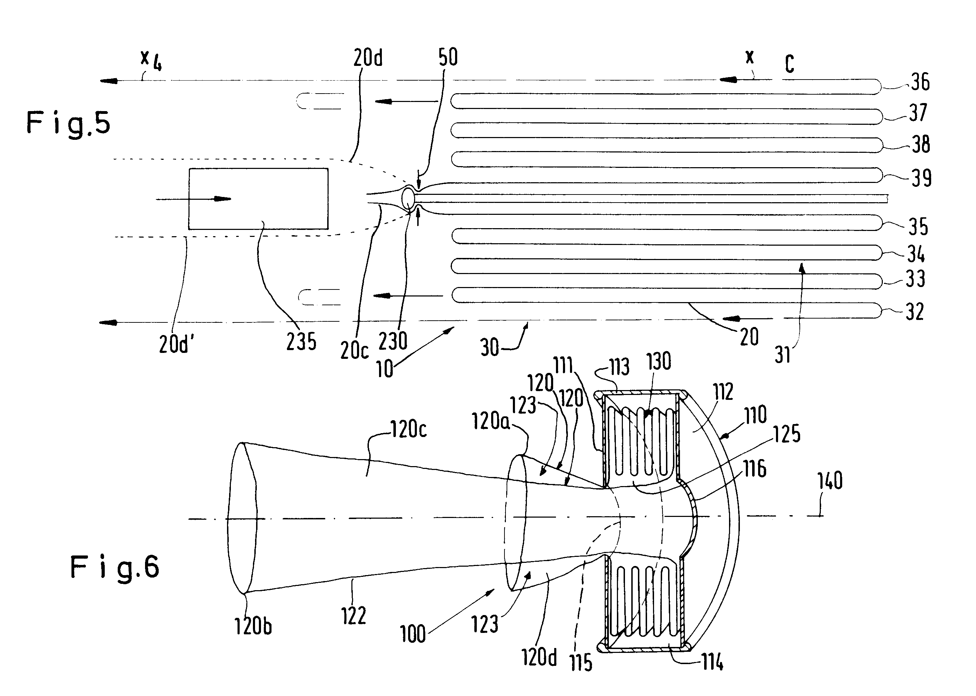

Der Einmal-Überzug 10 wird wie folgt verwendet:
In die Einführöffnung 41 zwischen den beiden Faltlagen 35,39 wird das Arthroskop 230 in Pfeilrichtung X3 eingeschoben und kommt im Innenraum des inneren Schlauchfolienabschnittes 20c zu liegen. Hat während des Einschiebens das Arthroskop 230 von der Stellung A die Stellung B eingenommen, dann wird hinter der Optik des Arthroskopes der innere Schlauchfolienabschnitt 20c vermittels eines Schlauchbinders 50 verschlossen, so daß das Arthroskop 230 einseitig umhüllt ist. Hieraufhin wird der äußere Schlauchfolienabschnitt 20d in Pfeilrichtung X aus der Stellung C abgezogen und über die zwischenzeitlich aufgesetzte Kamera gezogen, was in Pfeilrichtung X4 erfolgt. Ist die gesamte Schlauchfolie 20 von dem Schlauchfolienspeicher 30 abgezogen und somit alle Faltlagen aufgebraucht, dann stülpt sich der innere Schlauchfolienabschnitt 20c um das Arthroskop 230, was bei 20d′ angedeutet ist (Fig. 5).
Auf diese Weise sind sowohl das Arthroskop 230 als auch die Kamera 235 mittels einer zweifachen Folienlage abgedeckt.

In gleicher Weise wie der Einmal-Überzug 10 gehandhabt wird, wird auch der Einmal-Überzug 10a eingesetzt.

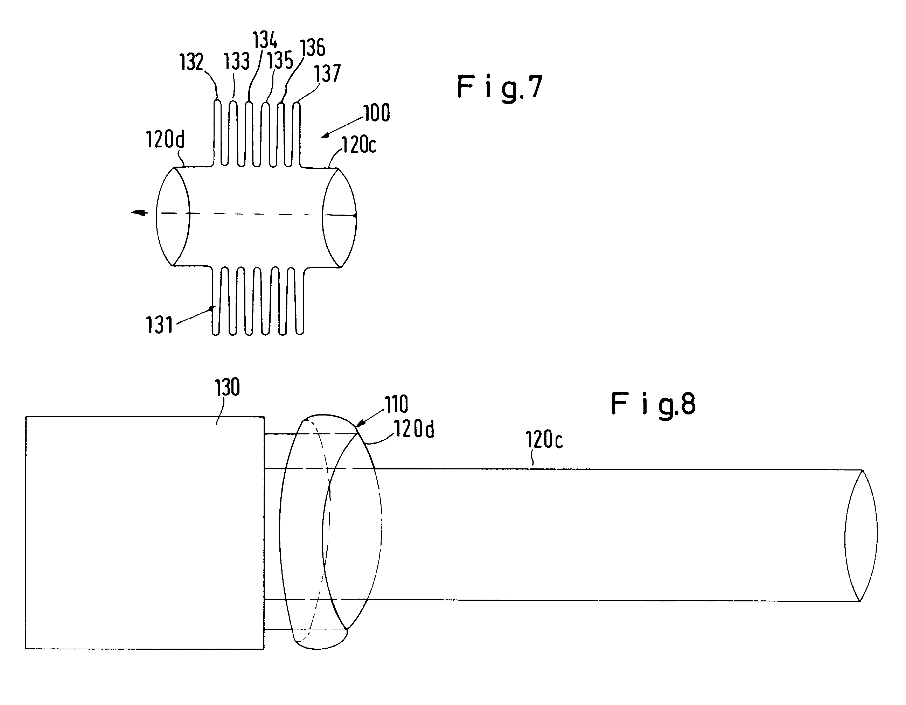

Nach Fig. 7 besteht der mit 100 bezeichnete Einmal-Überzug ebenfalls aus einer Schlauchfolie 120, wobei auch dieser Einmal-Überzug mit einem Schlauchfolienspeicher 130 versehen ist, der von einem Faltlagenpaket 131 aus einer Anzahl von Faltlagen 132,133,134, 135,136,137 gebildet wird, wobei gegenüber der Ausgestaltung des Einmal-Überzuges 10 bzw. 10a die Faltlagen 132 bis 137 quer zu der bei 140 in Fig. 6 angedeuteten Schlauchfolienlängsrichtung verlaufend sind, wohingegen die Faltlagen 32 bis 39 des Einmal-Überzuges 10 bzw. 10a parallel zur Schlauchfolienlängsrichtung 140 verlaufend sind (Fig.6). Beim Einmal-Überzug 100 werden die Faltlagen 132 bis 137 durch Raffung gebildet, wobei auch hier keine Festlegung auf sechs Faltlagen erfolgt. Die ziehharmonikaartige Faltung ist bei 131 angedeutet.

Bei dem Einmal-Überzug 100 ist der von den Faltlagen 132 bis 137 gebildete Schlauchfolienspeicher 130 in einem Behälter 110 derart angeordnet, daß ein innerer Schlauchfolienabschnitt 120c und ein äußerer Schlauchfolienabschnitt 120d gebildet wird, wobei die Schlauchfolienenden bei 120a,120b angedeutet sind. Der innere Schlauchfolienabschnitt 120c ist vor Einsatz des Einmal-Überzuges 100 gegenüber dem äußeren Schlauchfolienabschnitt 120d verlängert ausgebildet, so daß der verlängerte Abschnitt 122 des inneren Schlauchfolienabschnittes 120c aus dem Schlauchfolienende 120a des äußeren Schlauchfolienabschnittes 120d herausragt. Der kürzere Schlauchfolienabschnitt und somit der äußere Schlauchfolienabschnitt 120c ist bei 123 angedeutet.

Die Schlauchfolien 20,120 der Einmal-Überzüge 10,10a und 100 bestehen z.B aus Polyäthylen oder einem anderen geeigneten Kunststoff. Auch mit Kunststoffen beschichtete Papiere können,soweit diese sterilisierbar sind, ebenfalls eingesetzt werden.

Der den Schlauchfolienspeicher 130 aufnehmende Behälter 110 (Fig 6) ist scheibenförmig ausgebildet und besteht aus zwei sich gegenüberliegend angeordneten Wänden mit einer kreisförmigen Fläche und aus einer am Umfang der beiden Wände 111,112 umlaufenden Wand 113, so daß von den drei Wänden 111,112,113 der Behälterinnenraum 114 gebildet wird.

Jede Wand 111 bzw 112 des Behälters 110 ist mit einer mittigen Durchbrechung 115 bzw 116 versehen, wobei die beiden Durchbrechungen 115,116 deckungsgleich sind. Der Durchmesser der Durchbrechungen 115, 116 ist kleiner als der Durchmesser der Schlauchfolie 120. Die Durchbrechungen 115,116 sind vorzugsweise kreisförmig ausgebildet.

Die Schlauchfolie 120 ist,wie aus Fig. 7 ersichtlich, mit einer Faltung versehen, die von den Faltlagen 132 bis 137 gebildet wird. Dieser Schlauchfolienspeicher 130 mit seinen Faltlagen 132 bis 137 liegt im Innenraum 114 des Behälters 110. Dabei ist die Anordnung der Faltlagen 132 bis 137 bzw. die Ausbildung der Faltung und auch die Anordnung der Schlauchfolie 120 derart, daß das der Wand 112 zugekehrte Ende des Faltlagenpaketes 131 durch den von den Faltlagen 132 bis 137 gebildeten Innenraum 125 und durch die Durchbrechung 115 in der Behälterwand 111 des Behälters 110 hindurchgeführt und soweit herausgezogen ist, daß ein langer innerer Schlauchfolienabschnitt 120c erhalten wird.

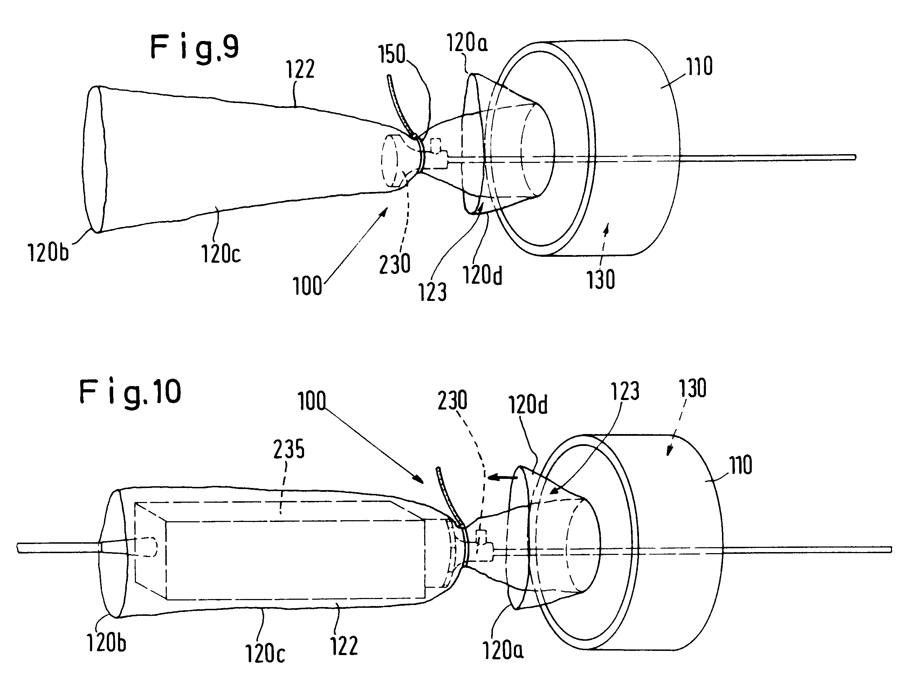

Das der Wand 111 des Behälters 110 zugekehrte Ende 120a der Schlauchfolie 120 ist aus der Durchbrechung 115 herausgeführt und liegt mit einem kurzen Abschnitt 120d um den längeren, inneren Schlauchfolienabschnitt 120c der Schlauchfolie 120 (Fig. 9). Auf diese Weise übergreift die Schlauchfolie 120 mit ihrem Ende 120a des kürzeren Schlauchfolienabschnittes 123 den inneren Schlauchfolienabschnitt 120c, der somit im Innenraum des äußeren Schlauchfolienabschnittes 120d liegt. Die im Bereich des Schlauchfolienendes 120a des äußeren Schlauchfolienabschnittes 120d liegende Öffnung ist bei 127 bezeichnet. Der Innenraum des längeren und inneren Schlauchfolienabschnittes 120c ist bei 125 angedeutet (Fig.6 und 9).

In Fig.9 ist das Arthroskop 230 angedeutet, auf das die Kamera 235 aufsetzbar ist. Mit 150 ist ein dem Schlauchbinder 50 des Einmal-Überzuges 10 bzw. 10a entsprechender Schlauchbinder bezeichnet.

Vor Gebrauch liegt der sterilisierte Einmal-Überzug, d.h. die Schlauchfolie 120 in dem Behälter 110, wobei die gesamte Einheit sterilisiert und steril verpackt ist, was auch auf den Einmal-Überzug 10 bzw. 10a zutrifft. Die beiden Schlauchfolienenden 120a, 120b ragen dabei aus der Durchbrechung 115 in der Wand 111 des Behälters 110 nur mit einem Griffabschnitt heraus. Bei der Anwendung des Einmal-Überzuges 100 wird zunächst das freie Ende 120b des Schlauchfolienabschnittes 120c erfaßt und aus dem Behälter 110 herausgezogen bis in etwa dieser Schlauchfolienabschnitt 120c die in Fig.6,9 und 10 gezeigte Länge aufweist. Das andere Schlauchfolienende 120a des äußeren Schlauchfolienabschnittes 120d wird nur geringfügig aus dem Innenraum 114 des Behälters 110 herausgezogen (Fig. 6).

Durch die Durchbrechung 115,116 in den Wänden 111,112 des Behälters 110 wird dann das Arthroskop 230 hindurchgeführt, wobei das Arthroskop 230 dann im Innenraum des inneren langen Schlauchfolienabschnittes 120c zu liegen kommt. Wie in Fig.9 dargestellt, erfolgt dann ein Verschließen der Schlauchfolie 20 im Bereich des Arthroskopes 30 vermittels des Schlauchbinders 150.

Hieraufhin wird dann in den Innenraum 125 des längeren, inneren Schlauchfolienabschnittes 120c die Kamera 235 eingeschoben und auf das Arthroskop 230 aufgesetzt. Weitere Schlauchbinder kommen dabei zusätzlich zur Anwendung, die sowohl das sterile Ende der Schlauchfolie 120 (Fig.9) als auch das unsterile Ende der Schlauchfolie 120 verschließen und zusätzlich in Schlauchmitte das schlauchumhüllte Kabel im Operationsfeld befestigen.

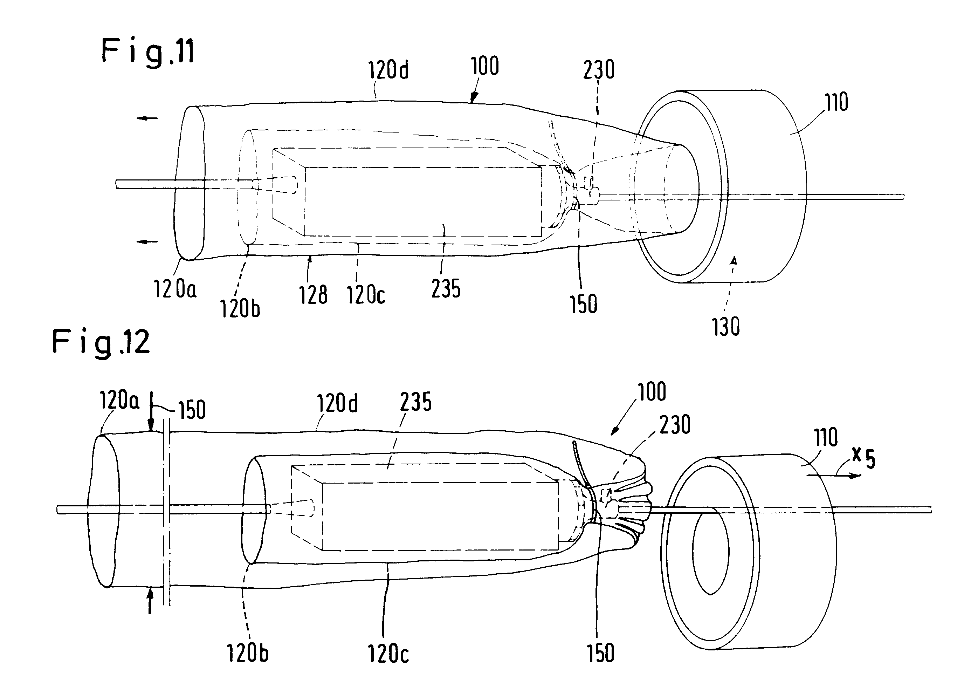

Ist die unsterile Kamera 235 in den Innenraum 125 des längeren,inneren Schlauchfolienabschnittes 120c eingeführt und auf das Arthroskop 230 aufgesetzt, wird der kurze äußere Schlauchfolienabschnitt 120d aus dem Behälter 110 unter Ausnutzung des gesamten Materialvorrates, der durch die Faltlagen 132 bis 137 gegeben ist, herausgezogen , und zwar bis über den die Kamera 235 umhüllenden Schlauchfolienabschnitt 128. Nach vollständiger Entfaltung der Schlauchfolie 120 und somit nach erfolgtem Aufbrauch des Schlauchfolienspeichers 130 bleibt der leere Behälter 110 zurück und die Kamera 235 mit dem Arthroskop 230 ist zweifach von Folie umhüllt (Fig.12). Das offene Ende des über den Schlauchfolienabschnitt 120c gezogenen Schlauchfolienabschnittes 120d wird dann vermittels eines Schlauchbinders verschlossen. Der leere Behälter 110 wird in Pfeilrichtung X5 abgezogen und nicht wieder verwendet. Der Behälter 110 kann mit einer Trennperforation versehen sein, um mühelos den Behälter zu öffnen und vom Arthroskop 230 abnehmen zu können.

Der Behälter 110 besteht aus Pappe, Karton oder anderen geeigneten Werkstoffen.

## Patentansprüche

1. Einmal-Überzug aus einer Schlauchfolie für Arthroskopiekameras, dadurch gekennzeichnet, daß der Überzug (10;10a;100) aus einer Schlauchfolie (20;120) mit einem Schlauchfolienspeicher (30;130) besteht, der
a) aus einem unter Ausbildung mehrerer, parallel zur Schlauchfolienlängsrichtung (40) verlaufenden, zu einem Faltlagenpaket (31) zusammengefaßten Faltlagen (32,33,34,35,36,37,38,39) mehrfach gefalteten Schlauchfolienabschnitt oder
b) aus einem unter Ausbildung mehrerer quer zur Schlauchfolienlängsrichtung (140) verlaufenden, zu einem Faltpaket (131) zusammengefaßten Faltlagen (132,133,134,135,136,137) mehrfach gerafften Schlauchfolienabschnitt
besteht, wobei die beiden Schlauchfolienenden (20a, 20b;120a,120b) eines jeden den Schlauchfolienspeicher (30;130) bildenden Schlauchfolienabschnittes auf dessen einer Seite und ineinanderliegend sind und wobei der jeweils innere Schlauchfolienabschnitt (20c;120c) mit einem Abschnitt (22,122) aus dem äußeren Schlauchfolienabschnitt (20d;120d) herausragend ist und benachbart zum Schlauchfolienende (20a; 220a)des Schlauchfolienabschnittes (23,123) einen Schlauchbinder (50;150) trägt.

2. Einmal-Überzug nach Anspruch 1, dadurch gekennzeichnet, daß der aus mehreren, parallel zur Schlauchfolienlängsrichtung (40) verlaufenden Faltlagen (32 bis 37) bestehende Überzug (10a) in beiden Endbereichen (31a,31b) des Faltlagenpaketes (31) liegende Schlauchfolienenden (20a,20b) aufweist, von denen das im Inneren des Faltlagenpaketes (31) liegende Schlauchfolienende (20a) in Richtung des äußeren Schlauchfolienabschnittes (20d) verlaufend ist.

3. Einmal-Überzug nach Anspruch 1, dadurch gekennzeichnet, daß der aus mehreren, parallel zur Schlauchfolienlängsrichtung verlaufenden Faltlagen (32 bis 39) bestehende Überzug (10a) in einem (31a) der beiden Endbereiche (31a,31b) des Faltlagenpaketes (31) liegende Schlauchfolienenden (20a,20b) aufweist.

4. Einmal-Überzug nach Anspruch 1 bis 3, dadurch gekennzeichnet, daß das Faltlagenpaket (31) des Überzuges (10;10a) vier und mehr Faltlagen (32 bis 39) aufweist.

5. Einmal-Überzug nach Anspruch 1, dadurch gekennzeichnet, daß das Faltlagenpaket (131) des aus mehreren quer zur Schlauchfolienlängsrichtung verlaufenden Faltlagen (132 bis 137) bestehenden Überzuges (100) in einem Behälter (110) angeordnet ist, dessen beide, sich gegenüberliegende Behälterwände (111,112) mit je einer mittigen Durchbrechung (115,116) versehen sind, und daß beide Durchbrechungen (115,116) sich gegenüberliegend und deckungsgleich zueinander angeordnet sind und einen Durchmesser aufweisen, der gegenüber dem Durchmesser der Schlauchfolie (120) kleiner bemessen ist, wobei die Schlauchfolie (120) in dem Innenraum (114) des Behälters (110) unter Ausbildung mehrerer Faltlagen (132 bis 137) in ziehharmonikaartiger Faltung (121) ihres größten Teils angeordnet und einendseitig mit einem kurzen Abschnitt (123) aus der Durchbrechung (115) in der Behälterwand (111) herausgeführt ist, wobei das andere Ende (120b) der Schlauchfolie (120) durch den Innenraum (125) der Schlauchfolie (120) im Bereich ihres gefalteten Abschnittes (121) hindurchgeführt und aus dem freien Ende (120a) des kurzen Schlauchfolienabschnittes (123) unter Ausbildung eines gegenüber dem kurzen Schlauchfolienabschnitt (123) länger bemessenen Schlauchfolienabschnitttes (122) herausgeführt ist, und daß der kürzere Schlauchfolienabschnitt (123) unter Ausnutzung des gefalteten Abschnittes (121) aus dem Innenraum (114) des Behälters (110) über den langen Schlauchabschnitt (122) hinausziehbar ausgebildet ist.

6. Einmal-Überzug nach Anspruch 5, dadurch gekenzeichnet, daß der Behälter (110) scheibenförmig mit einem kreisförmigen Durchmesser ausgebildet ist, wobei zwei sich gegenüberliegende Wände (111,112) und die die beiden Wände (111,112) miteinander verbindende umlaufende Außenwand (113) den Behälterinnenraum (114) bilden, und daß in den einandergegenüberliegenden Wänden (111,112) miteinander deckungsgleiche Durchbrechungen (115, 116) ausgebildet sind.

7. Verfahren zum Abdecken von Arthroskopiekameras mittels eines Einmal-Überzuges nach Anspruch 1 bis 4, dadurch gekennzeichnet, daß das freie Ende des außenliegenden Abschnittes einer zu einem Faltlagenpaket mit parallel zur Schlauchfolienlängsrichtung verlaufenden Faltlagen zusammengefalteten, sterilen Schlauchfolie um einen Abschnitt vom Faltlagenpaket abgezogen und über den innenliegenden, aus dem Faltlagenpaket geringfügig abgezogenen Abschnitt der Schlauchfolie hinweggezogen, hierauf in den Innenraum des innenliegenden Abschnittes der Schlauchfolie das Arthroskop eingeschoben, die Schlauchfolie im Bereich des freien Schlauchfolienendes hinter der Optik des Arthroskopes abgebunden, danach der außenliegende Abschnitt der Schlauchfolie unter Verwendung des Schlauchfolienvorrates des Faltlagenpaketes über die auf die Optik des Arthroskopes aufgesetzte Kamera bis sich der innenliegende Schlauchfolienabschnitt im Abbindungsbereich um die Optik des Arthroskopes stülpt zur Ausbildung einer zweilagigen Folienabdeckung gezogen und das freie Ende der abgezogenen Schlauchfolie hinter der Kamera abgebunden wird.

8. Verfahren zum Abdecken von Arthroskopiekameras mittels eines Einmal-Überzuges nach Anspruch 1,5 und 6, dadurch gekennzeichnet, daß das freie Ende des außenliegenden Abschnittes einer zu einem in einem geschlossenen Raum angeordneten Faltlagenpaket mit quer zur Schlauchfolienlängsrichtung verlaufenden Faltlagen zusammengefalteten sterilen Schlauchfolie um einen Abschnitt vom Faltlagenpaket abgezogen, der innenliegende Abschnitt der Schlauchfolie vom Faltlagenpaket mit einem gegenüber der Länge des äußeren Schlauchfolienabschnittes größeren Abschnitt abgezogen, hierauf in den Innenraum des inneren Schlauchfolienabschnittes das Arthroskop eingeschoben, die innere Schlauchfolie im Bereich der Optik des Arthroskopes abgebunden, danach in den inneren Schlauchfolienabschnitt durch dessen Öffnung am freien Ende die Kamera eingeschoben, darauf der äußere Schlauchfolienabschnitt unter Verwendung des Schlauchfolienvorrats des Faltlagenpaketes über die auf die Optik des Arthroskopes aufgesetzte Kamera und den inneren Schlauchfolienabschnitt bis sich der innenliegende Schlauchfolienabschnitt im Abbindungsbereich um die Optik des Arthroskopes stülpt zur Ausbildung einer zweilagigen Folienabdeckung gezogen und das freie Ende der abgezogenen Schlauchfolie hinter der Kamera abgebunden wird.

## Claims

1. Disposable thin-walled tube covering for an arthroscopy camera, characterized in that the covering (10;10a;100) comprises a thin-walled tube covering (20;120) with a storage means (30;130), which comprises
a) a repeatedly folded thin-walled tube section while forming several folding layers (32,33,34,35,36,37,38,39) that proceed parallel to the longitudinal direction of the thin-walled tube (40) and combined into a folding layer package (31, or
b) a repeatedly gathered thin-walled tube section while forming several folding layers (132,133,134,135,136, 137) proceeding transversely to the longitudinal direction of the thin-walled tube (140) and combined into a folding layer package (131),
wherein the two thin-walled tube ends (20a,20b;120a,120b) of each of the thin-walled tube sections forming the thin-walled tube storage means (30;130) are located on the one side of the same and disposed so as to repose within each other and in which the, in each case, internal thin-walled tube section (20c;120c) projects with a section (22,122) from the external thin-walled tube section (20d;120d) and, adjacent to the thin-walled tube end (20a;220a) of the thin-walled tube section (23,123) carries a tube tying means (50;150).

2. Disposable covering according to Claim 1, characterized in that the covering (10a) comprised of several folding layers (32 to 37) which proceed parallel to the longitudinal direction of the thin-walled tube (40), in that both terminal areas (31a,31b) of the folding layer package (31) are provided with thin-walled tube ends located therein (20a,20b), of which the thin-walled tube end (20a) located within the interior of the folding layer package (31) proceeds in the direction of the external thin-walled section (20d).

3. Disposable covering according to Claim 1, characterized in that the covering (10a) comprised of several folding layers (32 to 39) which proceed parallel to the longitudinal direction of the thin-walled tube, is, in one (31b) of the two terminal areas (31a,31b) of the folding layer package (31), provided with thin-walled tube ends (20a, 20b) which repose therein.

4. Disposable covering according to Claims 1 to 3, characterized in that the folding layer package (31) of the covering (10;10a) possesses four or more folding layers (32 to 39).

5. Disposable covering according to Claim 1, characterized in that the folding layer package (131) of the covering (100) comprising several folding layers (132 to 137) proceeding transversally to the longitudinal direction of the thin-walled tube is disposed in a container (110), whose two oppositely located container walls (111,112) are provided with one central perforation (115,116) each, and in that both perforations (115,116) are disposed so as to be located opposite each other as well as in full coincidence with each other and have a diameter which is smaller when compared with the diameter of the thin-walled tube (120), in which the thin-walled tube (120) is disposed in the interior (114) of the container (110) while forming several folding layers (132 to 137) in a a concertina-like folding (121 of its largest part and, on one end, with a short section (123), is passed out of the perforation (115) in the container wall (111), in which the other end (120b) of the thin-walled tube (120) is passed through the interior (125) of the thin-walled tube (120) within the area of its folded section (121) and is passed out from the free end (120a) of the short thin-walled tube section (123) while forming a thin-walled tube section (122) which, when compared with the short thin-walled tube section (123), is dimensioned so as to be longer, and in that the shorter thin-walled tube section (123), while utilizing the folded section (121), is constructed in such a way that it is possible for it to be drawn out from the interior (114) of the container (110) and over the long tube section (122).

6. Disposable covering according to Claim 5, characterized in that the container (110) is constructed in a disk-shaped fashion and possesses a circular diameter, wherein the oppositely located walls (111,112) and the outer wall (113) interconnecting the two walls (111,112) form the container interior (114), and in that, in the walls located opposite each other (111,112), perforations (115,116) are constructed which are in full coincidence with each other.

7. Method of covering arthroscopy cameras by means of a disposable covering according to the Claims 1 to 4, characterized in that the free end of the externally located section of a sterile thin-walled tube folded together so as to form a folding layer package, with folding layers that proceed parallel to the longitudinal direction of the thin-walled tube, is drawn from the folding layer package by one section and is drawn over the internally located section of the thin-walled tube which is drawn only insignificantly from the folding layer package, hereupon the arthroscope is inserted into the interior of the internally located section of the thin-walled tube, the thin-walled tube is tied off within the area of the free thin-walled tube behind the optics of the arthroscope, after this the externally located section of the thin-walled tube, while utilizing the supply of thin-walled tube from the folding layer package, is drawn over the camera mounted on the optics of the arthroscope until the internally located thin-walled tube section within the tying-off area is turned inside out around the optics of the athroscopy camera so as to form a two-layer tube sheet covering and the free end of the drawn-off thin-walled tube is tied off behind the camera.

8. Method for covering arthroscopy cameras by means of a disposable covering according to Claims 1, 5 and 6, characterized in that the free end of the externally located section of a sterile thin-walled tube folded together so as to form a folding layer package disposed within a closed space and having folding layers that proceed transversely to the longitudinal direction of the thin-walled tube, is drawn from the folding layer package by a section, the internally located section of the thin-walled tube is drawn from the folding layer package by a section which is greater than the length of the external thin-walled tube section, hereupon the arthroscope is inserted into the interior of the internal thin-walled tube section, the internal thin-walled tube is tied off within the area of the arthroscope optics, after this the camera is inserted into the internal thin-walled tube section through its opening on the free end, following this the external thin-walled tube section, while utilizing the supply of thin-walled tube of the folding layer package, is drawn over the camera mounted on the optics of the arthroscope until the internally located thin-walled tube section within the tying-off area is turned inside out around the optics of the arthroscope so as to form a two-layer tube sheet covering and the free end of the drawn-off thin-walled tube is tied off behind the camera.

## Revendications

1. Enveloppe à usage unique en feuille en gaine pour appareil photo à arthroscopie, **caractérisée en ce** que l'enveloppe (10 ; 10a ; 100) est constituée par une feuille en gaine (20 ; 120) avec un réservoir de feuille en gaine (30 ; 130) qui est constitué
a) par une portion de feuille en gaine pliée plusieurs fois en formant plusieurs couches de plis (32, 33, 34, 35, 36, 37, 38, 39) parallèles au sens longitudinal de la feuille en gaine (40), réunies en un paquet de couches de plis (31) ou
b) par une portion de feuille en gaine drapée plusieurs fois en formant plusieurs couches de plis (132, 133, 134, 135, 136, 137) transversales par rapport au sens longitudinal de la feuille en gaine (140), réunies en un paquet de plis (131),
les deux extrémités de la feuille en gaine (20a, 20b ; 120a, 120b) de chaque portion de feuille en gaine qui forme le réservoir de feuille en gaine (30 ; 130) se trouvant sur l'un des côtés de celui-ci et l'une dans l'autre et la portion de feuille en gaine respectivement intérieure (20c ; 120c) faisant saillie avec une portion (22, 122) de la portion extérieure de feuille en gaine (20d ; 120d) et portant un collier de serage (50 ; 150) au voisinage de l'extrémité de feuille en gaine (20a ; 220a) de la portion de feuille en gaine (23, 123).

2. Enveloppe à usage unique selon la revendication 1, **caractérisée en ce** que l'enveloppe (10a) qui est constituée par plusieurs couches de plis (32 à 37) parallèles au sens longitudinal de laa feuille en gaine (40) présente, dans ses deux zones d'extrémité (31a, 31b) du paquet de couches de plis (31), des extrémités de feuille en gaine couchées (20a, 20b) parmi lesquelles l'extrémité de feuille en gaine (20a) qui est située à l'intérieur du paquet de couches de plis (31) est située dans le sens de la portion extérieure de feuille en gaine (20d).

3. Enveloppe à usage unique selon la revendication 1, **caractérisée en ce** que l'enveloppe (10a) qui est constituée par plusieurs couches de plis (32 à 39) parallèles au sens longitudinal de la feuille en gaine présente, dans l'une (31a) des deux zones d'extrémité (31a, 31b) du paquet de couches de plis (31), des extrémités de feuille en gaine couchées (20a, 20b).

4. Enveloppe à usage unique selon les revendications 1 à 3, **caractérisée en ce** que le paquet de couches de plis (31) de l'enveloppe (10 ; 10a) présente quatre couches de plis ou davantage (32 à 39).

5. Enveloppe à usage unique selon la revendication 1, **caractérisée en ce** que le paquet de couches de plis (131) de l'enveloppe (100) qui est constituée par plusieurs couches de plis (132 à 137) transversales par rapport au sens longitudinal de la feuille en gaine est placé dans un conteneur (110) dont les deux parois de conteneur (111, 112) opposées l'une à l'autre sont pourvues d'une découpure centrale (115, 116) chacune et que les deux découpures (115, 116) sont placées en face l'une de l'autre et coincident l'une avec l'autre et qu'elles ont un diamètre qui est plus petit que le diamètre de la feuille en gaine (120), la feuille en gaine (120) étant placée dans l'intérieur (114) du conteneur (110) en formant plusieurs couches de plis (132 à 137) en pliage de type harmonica (121) de sa plus grande partie et qu'elle sort à l'une de ses extrémités avec une courte portion (123) de la découpure (115) de la paroi du conteneur (111), l'autre extrémité (120b) de la feuille en gaine (120) traversant l'espace intérieur (125) de la feuille en gaine (120) dans la zone de sa portion pliée (121) et sortant de l'extrémité libre (120a) de la courte portion de feuille en gaine (123) en formant une portion de feuille en gaine (122) qui est plus longue que la courte portion de feuille en gaine (123) et que la portion de feuille en gaine la plus courte (123) est configurée en pouvant être tirée hors de l'intérieur (114) du conteneur (110) sur la portion de gaine longue (122) en se servant de la portion pliée (121).

6. Enveloppe à usage unique selon la revendication 5, **caractérisée en ce** que le conteneur (110) est configuré en forme de disque avec un diamètre circulaire, deux parois opposées (111, 112) et la paroi extérieure (113) périphérique qui relie les deux parois (111, 112) l'une à l'autre formant l'intérieur du conteneur (114) et que des découpures (115, 116) qui coïncident l'une avec l'autre étant configurées dans les parois opposées l'une à l'autre (111, 112).

7. Procédé pour recouvrir des appareils photo pour arthroscopie au moyen d'une enveloppe à usage unique selon les revendications 1 à 4, **caractérisé en ce** que l'extrémité libre de la portion située à l'extérieur d'une feuille en gaine stérile pliée en un paquet de couches de plis avec des couches de plis parallèles au sens longitudinal de la feuille en gaine étant tirée d'une portion du paquet de couches de plis et étant tirée sur la portion située à l'intérieur qui est un peu retirée du paquet de couches de plis et étant ensuite glissée dans l'intérieur de la portion située à l'intérieur de la feuille en gaine de l'arthroscope, que la feuille en gaine est détachée dans la zone de l'extrémité libre de la feuille en gaine derrière l'objectif de l'arthroscope, que la portion située à l'extérieur de la feuille en gaine est ensuite tirée sur l'appareil photo posé sur l'objectif de l'arthroscope, en se servant des réserves de feuille en gaine du paquet de couches de plis, jusqu'à ce que la portion de feuille en gaine située à l'intérieur se retourne pour recouvrir l'objectif de l'arthroscope dans la zone où a lieu le détachement en formant un recouvrement de feuille en deux couches, et que l'extrémité libre de la feuille en gaine qui est tirée est détachée derrière l'appareil photo.

8. Procédé pour recouvrir des appareils photo pour arthroscopie au moyen d'une enveloppe à usage unique selon les revendications 1, 5 et 6, **caractérisé en ce** que l'extrémité libre de la portion située à l'extérieur d'une feuille en gaine stérile pliée en un paquet de couches de plis, qui est placé dans un espace fermé, avec des couches de plis transversales par rapport au sens longitudinal de la feuille en gaine est tirée d'une portion du paquet de couches de lis, que la portion située à l'intérieur de la feuille en gaine est tirée du paquet de couches de plis avec une portion plus grande que la longueur de la portion extérieure de feuille en gaine, que l'arthroscope est ensuite glissé dans l'intérieur de la portion intérieure de feuille en gaine, que la feuille en gaine intérieure est détachée dans la zone de l'objectif de l'arthroscope, que l'appareil photo est ensuite introduit dans la portion intérieure de feuille en gaine par son ouverture à l'extrémité libre, que la portion extérieure de feuille en gaine est ensuite tirée sur l'appareil photo posé sur l'objectif de l'arthroscope et sur la portion intérieure de feuille en gaine, en utilisant les réserves de feuille en gaine du paquet de couches de plis, jusqu'à ce que la portion intérieure de feuille en gaine se retourne, dans la zone où a lieu le détachement, pour recouvrir l'objectif de l'arthroscope pour former un recouvrement de feuille en deux couches, et que l'extrémité libre de la feuille en gaine tirée est détachée derrière l'appareil photo.
